# EUROPEAN PATENT APPLICATION

(11) **EP 2 666 465 A1**
(43) Date of publication of application: **27.11.2013**
(21) Application number: 12382211.6
(22) Date of filing: 25.05.2012
(51) Int. Cl.: A61K 31/46, A61M 15/00, A61P 11/06, A61P 11/08, A61K 31/573

(54) **Novel dosage and formulation**

(71) Applicant: Almirall, S.A., 08022 Barcelona (ES)
(72) Inventor: Lamarca Casado, Rosa, 08980 Sant Feliu de Llobregat (ES); De Miquel Serra, Gonzalo, 08980 Sant Feliu de Llobregat (ES)
(74) Representative: Elzaburu Marquez, Alberto

(57) **Abstract**

A pharmaceutical composition for inhalation comprising aclidinium in the form of a dry powder of a pharmaceutically acceptable salt in admixture with a pharmaceutically acceptable dry powder carrier, providing a delivered dose of aclidinium equivalent to about 322 micrograms aclidinium free base.

## Description

This invention relates to a novel dosage for aclidinium and to novel methods and formulations for the treatment of respiratory diseases, especially asthma and chronic obstructive pulmonary disease (COPD), using aclidinium.

### BACKGROUND

Aclidinium bromide is 3(R)-(2-hydroxy-2,2-dithien-2-ylacetoxy)-1-(3-phenoxypropyl)-1-azoniabicyclo[2.2.2]octane bromide, described in, e.g., WO 0104118. This compound is known to be a long-acting anticholinergic useful in the treatment of respiratory diseases.

### SUMMARY OF THE INVENTION

It is now surprisingly found that, for treatment of respiratory disorders, particularly asthma and COPD, in an adult human, aclidinium is most effective upon administration by inhalation in a dosage of about 322 micrograms (µg) delivered dose (weight corresponding to aclidinium free base), and/or a fine particle dose equivalent to about 140 µg aclidinium bromide. Typically the dose is a single dose or a twice daily dose, preferably a twice daily dose.

Typically, a delivered dose of 322 µg aclidinium free base corresponds to a dose of about 375 µg aclidinium bromide.

The invention thus provides in a first embodiment a pharmaceutical composition for inhalation comprising aclidinium in the form of a dry powder of a Pharmaceutical acceptable salt, e.g., aclidinium bromide, in admixture with a pharmaceutically acceptable dry powder carrier, e.g., lactose particles, (i) providing a delivered dose of aclidinium equivalent to about 322 µg aclidinium and/or a fine particle dose equivalent to about 140 µg aclidinium bromide, or (ii) in a multidose dry powder inhaler device calibrated to provide a delivered dose of aclidinium equivalent to about 322 µg aclidinium and/or a fine particle dose equivalent to about 140 µg aclidinium bromide. This composition can be administered one or more times per day. Preferably once or twice a day.

In a second embodiment, the invention provides a method of treating a respiratory condition, e.g., selected from asthma and chronic obstructive pulmonary disease, in a patient in need of such treatment, comprising administering a dose, typically a single daily dose or twice daily dose, of aclidinium, e.g., aclidinium bromide, equivalent to about 322 µg aclidinium and/or a fine particle dose equivalent to about 140 µg aclidinium bromide, e.g., comprising administering a pharmaceutical composition according to the previous paragraph. The invention further provides the use of aclidinium in the manufacture of a medicament, e.g., as described in the preceding paragraph, for use in such a method.

The aclidinium may be administered as monotherapy, or in combination with one or more additional anti-inflammatory and/or bronchodilating agents, e.g., corticosteroids, PDE IV inhibitors and β2-agonists, e.g., formoterol, salmeterol, budesonide, and mometasone, and the invention thus further provides methods as described above further comprising administration of an effective amount of such an agent, as well as pharmaceutical compositions as described above, further comprising such additional agent(s).

### DETAILED DESCRIPTION OF THE INVENTION

Typically, the aclidinium is administered in the form of a salt with an anion X, wherein X is a Pharmaceutical acceptable anion of a mono or polyvalent acid. More typically, X is an anion derived from an inorganic acid, such as hydrochloric acid, hydrobromic acid, sulphuric acid and phosphoric acid, or an organic acid such as methanesulphonic acid, acetic acid, fumaric acid, succinic acid, lactic acid, citric acid or maleic acid. Preferably the aclidinium is in the form of aclidinium bromide.

The aclidinium is preferably administered in the form of a dry powder, in admixture with a suitable carrier, e.g., lactose powder, suitable for inhalation.

For example, in one embodiment, the aclidinium is aclidinium bromide in admixture with lactose powder.

The respiratory disease or condition to be treated with the formulations and methods of the present invention is typically asthma, acute or chronic bronchitis, emphysema, chronic obstructive pulmonary disease (COPD), bronchial hyperreactivity or rhinitis, in particular asthma or chronic obstructive pulmonary disease (COPD), especially COPD.

In the context of dosage of an active agent, "about" as used herein means within the normal limits of acceptable variations as defined by the European and US Pharmacopeia of plus/minus 35% or preferably acceptable variations as defined by the current most stringent requirement, the US FDA draft guidance for inhaler of plus/minus 25%, or more preferably according to the CHMP Guideline on the Pharmaceutical Quality of Inhalation and Nasal Products of plus/minus 15%, and especially within the metered dosing accuracy for the dispensing system e.g. plus/minus 10%. Thus a delivered dose of "about 322 µg aclidinium free base" is meant a target dose of 322 µg aclidinium subject to variation within the normal limits of acceptance for the dispensing system, e.g. 209-435 µg aclidinium (plus/minus 35%, acceptable variations as defined by the European and US Pharmacopeia) or preferably 241-403 µg aclidinium (plus/minus 25%, acceptable variations as defined by the current most stringent requirement, the US FDA draft guidance for inhaler), or more preferably 273-371 µg aclidinium (plus/minus 15%, acceptable variations defined by the CHMP Guideline on the Pharmaceutical Quality of Inhalation and Nasal Products) or especially 289-355 µg aclidinium (or within the metered dosing accuracy of the inhaler).

A delivered dose of "about 375 µg aclidinium bromide" is meant a target dose of 375 µg aclidinium bromide subject to variation within the normal limits of acceptance for the dispensing system, e.g. 242-507 µg aclidinium bromide (plus/minus 35%, acceptable variations as defined by the European and US Pharmacopeia) or preferably 281-469 µg aclidinium bromide (plus/minus 25%, acceptable variations as defined by the current most stringent requirement, the US FDA draft guidance for inhaler), or more preferably 319-431 µg aclidinium bromide (pluslminus 15%, acceptable variations defined by the CHMP Guideline on the Pharmaceutical Quality of Inhalation and Nasal Products) or especially 337-413 µg aclidinium bromide (or within the metered dosing accuracy of the inhaler).

The fine particle dose (fine particle dose = µg aclidinium bromide in the delivered dose below a cut off aerodynamic threshold of 5 micrometer) are also subjected to variations. Therefore, a fine particle dose of "about 140 µg aclidinium bromide" is meant a target dose of 79-206 µg aclidinium bromide, preferably 100-190 µg aclidinium bromide, more preferably 110-180 µg aclidinium bromide.

Packaging of the formulation may be suitable for unit dose or multi-dose delivery. In the of multi-dose delivery, the formulation can be pre-metered or metered in use. Dry powder inhalers are thus classified into three groups: (a) single dose, (b) multiple unit dose and (c) multi dose devices.

Formulations generally contain a powder mix for inhalation of the compounds of the invention and a suitable powder base (carrier substance) such as lactose or starch. Use of lactose is preferred. Each capsule or cartridge may generally contain between 2µg and 400 µg of each therapeutically active ingredient. Alternatively, the active ingredient (s) may be presented without excipients.

For single dose inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients. Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported.

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (e. g. EP0069715) or disks (e. g. GB 2041763; EP 0424790; DE 4239402 and EP 0674533), rotatable cylinders (e. g. EP 0166294; GB 2165159 and WO 92/09322) and rotatable frustums (e. g. WO 92/00771), all having cavities which have to be filled with powder from the container. Other multi dose devices have measuring slides (e. g.US 5201308 and WO 97/00703) or measuring plungers with a local or circumferential recess to displace a certain volume of powder from the container to a delivery chamber or an air conduit e. g. EP 0505321, WO 92/04068 and WO 92/04928.

Reproducible dose measuring is one of the major concerns for multi dose inhaler devices. The powder formulation has to exhibit good and stable flow properties, because filling of the dose measuring cups or cavities is mostly under the influence of the force of gravity. For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (e. g. WO 97/000703). For such semi-permanent multi dose inhalers with disposable drug containers, the requirements to prevent drug accumulation are even stricter.

In a preferred embodiment, the aclidinium is administered via a breath-activated, multidose, dry powder inhaler, calibrated to permit daily dosing of 400 µg metered nominal dose of aclidinium. An especially preferred inhaler device for this purpose is Genuair®, (formerly known as Novolizer SD2FL), or as described in WO 97/000703, WO 03/000325, or WO 03/061742, the contents of which applications are incorporated herein by reference.

Apart from applications through dry powder inhalers the compositions of the invention can be administered in aerosols which operate via propellant gases or by means of so-called atomisers or nebulizers, via which solutions or suspensions of pharmacologically-active substances can be sprayed under high pressure so that a mist of inhalable particles results.

Medicaments for administration by inhalation desirably have a controlled particle size. The optimum particle size for inhalation into the bronchial system is usually 1-10µm, preferably 2-5µm. Particles having a size above 20µ are generally too large when inhaled to reach the small airways. To achieve these particle sizes the particles of the active ingredient as produced may be reduces by conventional means eg by micronisation or supercritical fluid techniques. The desired fraction may be separated out by air classification or sieving. Preferably, the particles will be crystalline.

Achieving a high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient, for example a mono-, di- or polysaccharide or sugar alcohol, e.g., such as lactose, mannitol or glucose is generally employed. The particle size of the excipient will usually be much greater than the inhaled medicament within the present invention. When the excipient is lactose it will typically be present as lactose particles, preferably crystalline alpha lactose monohydrate, e.g., having an average particle size range of 20-1000 µ.m, preferably in the range of 90-150 µm. The median particle size approximately corresponds to the average and is the diameter where 50 mass-% of the particles have a larger equivalent diameter, and the other 50 mass-% have a smaller equivalent diameter. Hence the average particle size is generally referred to in the art as equivalent d50. The distribution of particle size around may affect flow properties, bulk density, etc. Hence to characterize a particle diameter, other equivalent diameters can be used in addition to d50, such as d10 and d90. d10 is the equivalent diameter where 10 mass-% of the particles have a smaller diameter (and hence the remaining 90% is coarser). d90 is the equivalent diameter where 90 mass-% of the particles have a smaller diameter. In one embodiment, the lactose particles for use in formulations of the invention have a d10 of 90 - 160 µm, a d50 of 170 - 270 µm, and d90 of 290 - 400 µm.

Suitable lactose maternal for use in the present invention are commercially available, e.g., from DMW lnternacional (Respitose GR-001, Respitose SV-001, Respitose SV-003); eggle (Capsulac 60, lnhalac 70, Capsulac 60 INH); and Borculo Domo (Lactohale 100-200, Lactohale 200-300, and Lactohale 100-300).

The ratio between the lactose particles and the aclidinium by weight will depend on the inhaler device used, but is typically, e.g., 5:1 to 100:1, for example 25:1 to 75:1, preferably 25:1 to 50:1, more preferably 30-35:1.

In a preferred embodiment, the aclidinium is administered in the form of a dry powder formulation of aclidinium bromide in admixture with lactose, in a ratio by weight of aclidinium to lactose of 1:25 to 1:50, suitable for administration via a dry powder inhaler, wherein the aclidinium particles have an average particle size of from 2 to 5µm in diameter, e.g., less than 3 µm in diameter, and the lactose particles have have a d10 of 90 - 160 µm, a d50 of 170 - 270 µm, and d90 of 290 - 400 µm.

Additional active agents such as β2-agonists , PDE IV inhibitors, corticosteroids, leukotriene D4 antagonists, inhibitors of egfr-kinase, p38 kinase inhibitors or NK1 receptor agonists may be utilized in the methods and formulations of the inventions. For example, the invention provides aclidinium formulations as described herein further comprising an effective amount of one or more such additional active agents, e.g. further comprising an effective amount of a β2-agonist and/or a PDE IV inhibitor and/or a corticosteroid. The invention also provides methods for treating respiratory conditions as herein before described, e.g., asthma or COPD, comprising administering an aclidinium formulation as described herein and further comprising administering simultaneously an effective amount of one or more such additional active agents, e.g. further comprising an effective amount of a β2-agonist and/or a PDE IV inhibitor and/or a corticosteroid.

β2-agonists suitable for use with the aclidinium in the present invention include, e.g., arformoterol, bambuterol, bitolterol, broxaterol, carbuterol, clenbuterol, dopexamine, fenoterol, formoterol, hexoprenaline, ibuterol, isoetharine, isoprenaline, levosalbutamol, mabuterol, meluadrine, metaprotenerol, nolomirole, orciprenaline, pirbuterol, procaterol, reproterol, ritodrine, rimoterol, salbutamol, saimefamol, saimeterol, sibenadet, sotenerot, sulfonterol, terbutaline, tiaramide, tulobuterol, GSK-597901, milveterol, GSK-678007, GSK-642444, GSK-159802, HOKU-81, abediterol (LAS100977), KUL-1248, carmoterol, indacaterol and 5-[2-(5,6-diethylindan-2-ylamino)-1-hydroxyethyl]-8-hydroxy-1H-quinolin-2-one, 4-hydroxy-7-[2-1[2-([3-(2-phenylethoxy)propyl]sulfonyll ethyl]aminolethyl]-2(3H)-benzothiazolone, 1-(2-fluoro-4-hydroxyphenyl)-2-[4-(1-benzimidazolyl)-2-methyl-2-butylamino]ethanol, 1 -[3-(4-methoxybenzylamino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N -dimethylaminophenyl)-2-methyl-2-propylamino]ethanol, 1-[2H-5-hydroxy-3-oxo-4H-1,4-benzoxazin-8-yl]-2-[3-(4-methoxypheny))-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1 -[2H-5-hydroxy-3-oxo-4H-1,4-benzoxazin-8-y)]-2-{4-[3-(4-methoxyphenyl)-1,2,4-triazo)-3-yl]-2-methyl-2-butylamino}ethano), 5-hydroxy-8-(1-hydroxy-2-isopropylaminobutyl)-2H-1,4-benzoxazin-3-(4H)-one, 1-(4-amino-3-chloro-5-trifluoromethylphenyl)-2-tert-butylamino)ethanol and 1-(4-ethoxycarbonylamino-3-cyano-5-fluorophenyl)-2-(tert-butylamino)ethanol optionally in the form of their racemates, their enantiomers, their diastereomers, and mixtures thereof, and optionally their pharmacologically-compatible acid addition salts.

The preferred β2-agonists to be used in the combinations of the invention are: arformoterol, bambuterol, bitolterol, broxaterol, carbuterol, clenbuterol, dopexamine, fenoterol, formoterol, hexoprenaline, ibuterol, isoprenaline, levosalbutamol, mabuterol, meluadrine, nolomirole, orciprenaline, pirbuterol, procaterol, (R,R)-formoterol, reproterol, ritodrine, rimoterol, salbutamol, salmeterol, sibenadet, sulfonterol, terbutaline, tulobuterol, GSK-597901, milveterol, abediterol (LAS100977), KUL-1248, carmoterol and indacaterol optionally in the form of their racemates, their enantiomers, their diastereomers, and mixtures thereof, and optionally their pharmacologically-compatible acid addition salts.

Since the M3 antagonists of the invention have a long duration of action, it is preferred that they are combined with long-acting β2-agonists (also known as LABAs). The combined drugs could thus be administered once or twice a day.

Particularly preferred LABAs are formoterol, salmeterol and GSK-597901, milveterol, LAS100977 (5-(2-{[6-(2,2-difluoro-2-phenylethoxy)hexyl]amino}-1*(R)*-hydroxyethyl)-8-hydroxyquinolin-2(1*H*)-one), KUL-1248, carmoterol and indacaterol optionally in the form of their racemates, their enantiomers, their diastereomers and mixtures thereof, and optionally their pharmacologically-compatible acid addition salts. More preferred are salmeterol, formoterol, abediterol (LAS100977), and indacaterol. Still more preferred are salmeterol, formoterol and LAS100977 (5-(2-{[6-(2,2-difluoro-2-phenylethoxy)hexyl]amino}-1*(R)*-hydroxyethyl)-8-hydroxyquinolin-2(1*H*)-one), in particular salmeterol xinafoate,formoterol fumarate and LAS100977 (5-(2-{[6-(2,2-difluoro-2-phenylethoxy)hexyl]amino}-1*(R)*-hydroxyethyl)-8-hydroxyquino)in-2(1*H*)-one).

For example, the invention provides a pharmaceutical composition for inhalation comprising aclidinium in the form of a dry powder of a Pharmaceutical acceptable salt, e.g., bromide, in admixture with a pharmaceutically acceptable carrier, e.g., lactose particles, together with formoterol fumarate, (i) providing a delivered dose of aclidinium equivalent to about 322 µg aclidinium free base and/or a fine particle dose equivalent to about 140 µg aclidinium bromide together with a single metered nominal dose of about 5-25 µg (e.g. 6, 8.5, 12, 18 or 24 µg, for example 6 or 12 µg) formoterol fumarate or (ii) in a multidose dry powder inhaler device calibrated to provide a delivered dose of aclidinium equivalent to about 322 µg aclidinium free base and/or a fine particle dose equivalent to about 140 µg aclidinium bromide together with a metered nominal dose of about 5-25 µg (e.g. 6, 8.5,12, 18 or 24 µg, for example 6 or 12 µg) formoterol fumarate. A metered nominal dose of about 6 µg formoterol fumarate typically corresponds to a delivered dose of 4.5 µg formoterol fumarate and a metered nominal dose of about 12 µg formoterol fumarate typically corresponds to a delivered dose of 9 µg formoterol fumarate.

The pharmaceutical composition for inhalation comprising aclidinium and a β2-agonist, for example, formoterol or abediterol (LAS100977), can be administered one or more times per day. Preferably once or twice a day.

Examples of suitable PDE4 inhibitors that can be combined with aclidinium in the present invention are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, 6-[2-(3,4-Diethoxyphenyl)thiazol-4-yl]pyridine-2-carboxylic acid (tetomilast), (R)-(+)-4-[2-(3-Cyclopentyloxy-4-methoxyphenyl)-2-phenylethyl]pyridine (CDP-840), N-(3,5-Dichloro-4-pyridinyl)-2-[l-(4-fluorobenzyl)-5-hydroxy-1H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide (D-4418), N-[9-Methyl-4-oxo-1-phenyl-3,4,6,7-tetrahydropyrrolo[3,2,1-jk][1,4]benzodiazepin-3(R)-yl]pyridine-4-carboxamide, 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsulfonyl]-4-(3-methoxyphenylamino)-8-methylquinoline-3-carboxamide hydrochloride (GSK-256066), 4-[6,7-Diethoxy-2,3-bis(hydroxymethyl)naphthalen-1-yl]-1-(2-methoxyethyl)pyridin-2(1H)-one (T-440), (-)-trans-2-[3'-[3-(N-Cyclopropylcarbamoyl)-4-oxo-1,4-dihydro-1,8-naphthyridin-1-yl]-3-fluorobiphenyl-4-yl]cyclopropanecarboxylic acid (MK-0873), CDC-801, UK-500001, BLX-914, 2-carbomethoxy-4-cyano-4-(3-cyclopropylmethoxy-4-difluroromethoxyphenyl)cyclohexanl -one, *cis* [4-cyano-4-(3-cyclopropy)methoxy-4-difluoromethoxyphenyl)cyclohexan-1-ol, 5(S)-[3-(Cyclopentyloxy)-4-methoxyphenyl]-3(S)-(3-methylbenzyl)piperidin-2-one (IPL-455903), ONO-6126 (Eur Respir J 2003, 22(Suppl. 45): Abst 2557) and the compounds claimed in the PCT patent applications number WO03/097613, W02004/058729, WO 2005/049581, WO 2005/123693 and WO 2005/123692.

Examples of suitable corticosteroids and glucocorticoids that can be combined with aclidinium in the present invention are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, Butixocort propionate, RPR-106541, halometasone, methylprednisolone suleptanate, mometasone furoate, rimexolone, prednisolone farnesylate, ciclesonide, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11 beta-hydroxy-17alpha-[2-(methylsulfanyl)acetoxy]-4-pregnene-3,20-dione, Desisobutyrylciclesonide, hydrocortisone acetate, hydrocortisone sodium succinate, NS-126, prednisolone sodium phosphate, hydrocortisone probutate, prednisolone sodium metasulfobenzoate and clobetasol propionate, especially budesonide or mometasone.

For example, the invention provides a pharmaceutical composition for inhalation comprising aclidinium in the form of a dry powder of a pharmaceutically acceptable salt, e.g., bromide, in admixture with a Pharmaceutical acceptable carrier, e.g., lactose particles, together with mometasone furoate, (i) providing a delivered dose of aclidinium equivalent to about 322 µg aclidinium free base and/or a fine particle dose equivalent to about 140 µg aclidinium bromide together with a single metered nominal dose of about 100-900 µg (e.g. , 100, 110, 200, 220, 300, 330, 400, 440, 800 or 880 µg, for example 200-450, e.g 220 or 440 µg) mometasone furoate, or (ii) in a multidose dry powder inhaler device calibrated to provide a delivered dose of aclidinium equivalent to about 322 µg aclidinium free base and/or a fine particle dose equivalent to about 140 µg aclidinium bromide together with a metered nominal dose of about 100-900 µg (e.g. 100, 110, 200, 220, 300, 330, 400, 440, 800 or 880 µg, for example 200-450, e.g 220 or 440 µg) mometasone furoate.

The pharmaceutical composition for inhalation comprising aclidinium and a corticosteroid, for example mometasone furoate, can be administered one or more times per a day. Preferably once or twice a day.

The invention also provides a pharmaceutical composition comprising aclidinium, a β2-agonist as defined above and a corticosteroid, as defined above. Most preferred β2-agonists are selected from abediterol (LAS100977) and formoterol. Most preferred corticosteroid is a mometasone furoate. These triple combinations are suitable for administration once or twice a day.

## Claims

1. A pharmaceutical composition for inhalation comprising aclidinium in the form of a dry powder of a Pharmaceutical acceptable salt in admixture with a pharmaceutical acceptable dry powder carrier, providing a delivered dose of aclidinium equivalent to about 322 µg aclidinium free base and/or a fine particle dose equivalent to about 140 µg aclidinium bromide.

2. A pharmaceutical composition according to claim 1, in the form of a single-dose dry powder formulation providing a delivered dose of aclidinium equivalent to about 322 µg aclidinium free base and/or a fine particle dose equivalent to about 140 µg aclidinium bromide.

3. A pharmaceutical composition according to claim 1 or claim 2, in the form of a multi-dose dry powder formulation for administration in a multidose dry powder inhaler device calibrated to provide a delivered dose of aclidinium equivalent to about 322 µg aclidinium free base and/or a fine particle dose equivalent to about 140 µg aclidinium bromide.

4. The pharmaceutical composition according to any one of the preceding claims wherein the pharmaceutically acceptable salt of aclidinium is aclidinium bromide.

5. The pharmaceutical composition according to any of the preceding claims wherein the Pharmaceutical acceptable carrier is lactose particles.

6. The pharmaceutical composition according to any of the preceding claims wherein the ratio by weight of aclidinium to carrier is from 1:25 to 1:75, preferably in the ratio from 1:25 to 1:50.

7. The pharmaceutical composition according to any of the preceding examples wherein the average particle diameter of the aclidinium is within 2-5µm.

8. The pharmaceutical composition according to any of the preceding examples wherein the carrier particles have a d10 of 90 - 160 µm, a d50 of 170 - 270 µm, and d90 of 290 - 400 µm.

9. The pharmaceutical composition according to any of the preceding claims further comprising an effective amount of one or more additional active agents selected from β2-agonists, PDE IV inhibitors, and corticosteroids.

10. The pharmaceutical composition according to claim 10 wherein the additional active agent is selected from formoterol, salmeterol, budesonide and mometasone, in free or pharmaceutically acceptable salt form.

11. The pharmaceutical composition according to claim 11 wherein the additional active agent is formoterol fumarate in an amount of about 5-25 µg per metered nominal dose.

12. The pharmaceutical composition according to claim 12 wherein the additional active agent is formoterol fumarate in an amount of about 6 µg per metered nominal dose.

13. The pharmaceutical composition according to claim 12 wherein the additional active agent is formoterol fumarate in an amount of about 12 µg per metered nominal dose.

14. A method of treating a respiratory condition selected from asthma and chronic obstructive pulmonary disease in a patient in need of such treatment, comprising administering a single daily delivered dose of aclidinium equivalent to about 322 µg aclidinium free base and/or a fine particle dose equivalent to about 140 µg aclidinium bromide.

15. A method of treating a respiratory condition selected from asthma and chronic obstructive pulmonary disease in a patient in need of such treatment, comprising administering twice daily delivered dose of aclidinium equivalent to about 322 µg aclidinium free base and/or a fine particle dose equivalent to about 140 µg aclidinium bromide.

16. The method of claim 15 or 16 comprising administering a pharmaceutical composition according to any of claims 1-14.

17. The method of any one of claims 15-17 further comprising administering an effective amount of one or more additional active agents selected from β2-agonists, PDE IV inhibitors, and corticosteroids.

18. The method of claim 18 wherein the additional active agent is selected from formoterol, salmeterol, budesonide, and mometasone in free or pharmaceutically acceptable salt form.

19. The method according to claim 19 wherein the additional active agent is formoterol fumarate in an amount of about 5-25 µg per metered nominal dose.

20. The use of aclidinium in free or Pharmaceutical acceptable salt form in the manufacture of a medicament for administration in accordance with the method of any of claims 15-20.

21. The use of aclidinium in free or pharmaceutically acceptable salt form in the manufacture of a pharmaceutical composition according to any of claims 1-14.

22. Aclidinium in free or pharmaceutically acceptable salt form for use in any of the methods of claims 15-20.

23. The formulation according to any of claims 1-14 for use in any of the methods of claims 15-20.

24. The formulation according to any of claims 1-14 for use in the treatment of a respiratory condition selected from asthma and chronic obstructive pulmonary disease.

25. A dry powder inhaler device calibrated to deliver, upon actuation, a delivered dose of aclidinium equivalent to about 322 µg aclidinium free base and/or a fine particle dose equivalent to about 140 µg aclidinium bromide.

26. A dry powder inhaler device according to claim 26, wherein the device is single-dose and/or a multi-dose.
